# EUROPÄISCHE PATENTSCHRIFT

(11) **EP 1 329 192 B1**
(45) Veröffentlichungstag und Bekanntmachung des Hinweises auf die Patenterteilung: **13.04.2005**
(21) Anmeldenummer: 02027953.5
(22) Anmeldetag: 13.12.2002
(51) Int. Cl.: A61B 6/00, A61B 6/06

(54) **Röntgeneinrichtung**
X-ray device
Dispositif de rayons X

(30) Priorität: 18.01.2002 DE 10201868
(43) Veröffentlichungstag der Anmeldung: 23.07.2003
(73) Patentinhaber: SIEMENS AKTIENGESELLSCHAFT, 80333 München (DE)
(72) Erfinder: Schmitt, Thomas, 91301 Forchheim (DE)

(56) Entgegenhaltungen:
- DE-A- 19 729 414
- DE-A- 19 832 973
- DE-U- 8 312 137

## Beschreibung

Die Erfindung betrifft eine Röntgeneinrichtung umfassend eine Strahlungsquelle, einen austauschbaren Strahlungsfilter und eine Flächendosismesseinrichtung umfassend eine Messkammer mit zugeordneter Auswerteeinrichtung zur Ermittlung des Flächendosisproduktes anhand der von der Messkammer gegebenen Messsignale, wobei die Messkammer bezogen auf die Strahlungsrichtung vor dem Filter im Strahlengang angeordnet ist.

Die Messung des Flächendosisproduktes dient dazu, die dem Untersuchungsobjekt, beispielsweise einem Patienten applizierte Röntgenstrahlungsmenge zu bestimmen. Hierzu dient eine Flächendosismesseinrichtung mit einer Messkammer, die im Strahlengang angeordnet ist. Bei dieser Messkammer handelt es sich in der Regel um eine Ionisationskammer, durch die die Röntgenstrahlung hindurchtritt und an der ein von der Strahlungsmenge abhängiges Ausgangssignal abgegriffen werden kann. Hierüber kann dann die applizierte flächenbezogene Dosis ermittelt werden, in der Regel in µGym²-Einheiten.

Häufig werden weiterhin bei bekannten Röntgeneinrichtungen Strahlungsfilter verwendet, um die Röntgenstrahlung in bestimmten filterspezifischen Bereichen zu schwächen oder gänzlich auszublenden. Dabei sind unterschiedlichste austauschbare und einsetzbare Filter bekannt, beispielsweise Schulterfilter, Fußfilter, Beckenfilter oder Schädelfilter. Diese Filter, die in der Regel in Form von im Wesentlichen rechteckigen Filterplatten ausgebildet sind, werden in den Strahlengang geschoben. Diese Einschubführungen sind in der Regel außerhalb des Gehäuses, in dem die Messeinrichtung und weitere Teile der Flächendosismesseinrichtung wie z.B. gegebenenfalls die Auswerteeinrichtung aufgenommen sind, angeordnet, das heißt die Messkammer liegt bezogen auf die Strahlungsrichtung vor dem Filter. Die Messkammer ist beispielsweise häufig in einer der Strahlungsquelle nachgeschalteten Blendeneinrichtung, insbesondere der Tiefenblende integriert, an deren äußerem Gehäuseabschnitt dann die entsprechenden Einschubführungen für die plattenartigen Strahlungsfilter wären. Eine solche Röntgeneinrichtung ist beispielsweise in der DE 83 12 137 U beschrieben.

Aus dieser Anordnung, nach welcher die Strahlungsfilter der Messkammer nachgeschaltet sind, resultiert jedoch das Problem, dass in das ermittelte Flächendosisprodukt die Filterwirkung nicht eingeht, das heißt, die Filterleistung, die die tatsächlich applizierte Röntgenstrahlung schwächt, wird nicht berücksichtigt, da die Messung vor dem Strahlungsfilter stattfindet. Die applizierte Dosis ist also im Endeffekt geringer als das "ungefilterte" Messergebnis angibt.

Der Erfindung liegt damit das Problem zugrunde, eine Röntgeneinrichtung anzugeben, die die eingangsgenannten Nachteile beseitigt.

Zur Lösung dieses Problems ist bei einer Röntgeneinrichtung der eingangsgenannten Art erfindungsgemäß vorgesehen, dass Mittel zum Erkennen der Art und/oder des Types des Filters vorgesehen sind, wobei die Auswerteeinrichtung zum Korrigieren des ermittelten Flächendosisproduktes anhand wenigstens eines filterspezifischen Korrekturwerts ausgebildet ist.

Bei der erfindungsgemäßen Röntgeneinrichtung ist es über die Erkennungsmittel möglich, festzustellen, welcher Filtertyp oder welche Filterart in den Strahlengang hinter der Messkammer geschoben wurde. Anhand dieser Kenntnis, die an die Auswerteeinrichtung gegeben wird, ist diese nun in der Lage, unter Verwendung wenigstens eines filterspezifischen Korrekturwertes, also eines Korrekturwertes, der die Filtereigenschaften des erkannten Filters berücksichtigt, das über die Messkammer vor dem Strahlungsfilter erfasste Flächendosisprodukt, das letztlich in der Auswerteeinrichtung tatsächlich berechnet wird, entsprechend zu korrigieren. Hierdurch ist es möglich, im Nachhinein die Filterwirkung des nachgeschalteten Strahlungsfilters mit in das errechnete Flächendosisprodukt einzurechnen, so dass dieses weitestgehend die tatsächlich applizierte Röntgenstrahlung und nicht die ungeschwächte Strahlungsmenge angibt.

Um die Art beziehungsweise den Typ des Filters zu erkennen ist es erforderlich, dass jeder eingeschobene oder einsetzbare Filter filterspezifisch Merkmale oder Kennzeichen aufweist, die in irgendeiner Weise erfasst werden können. In einer ersten Erfindungsausgestaltung können hierbei die Mittel zum Erkennen der Art und/oder des Typs des Filters wenigstens einen am Filter angeordneten Transponder und eine das Transpondersignal aufnehmende Erfassungseinrichtung umfassen. Diese Erfassungseinrichtung, die selbstverständlich gleichzeitig auch die entsprechende Anregungseinrichtung sein kann, die den Transponder zum Aussenden des Transpondersignals aktiviert, kann extern zur Flächendosismesseinrichtung sein, alternativ dazu kann auch die Auswerteeinrichtung selbst hierzu ausgebildet sein. Jeder Strahlungsfilter verfügt über eigenen filterspezifischen Transponder, so dass eine einfache Erfassung und Unterscheidung möglich ist.

Alternativ hierzu können die Mittel zum Erkennen der Art und/oder des Typs wenigstens eine am Filter vorgesehene, die Art und/oder den Typ des Filters angebende Kennzeichnung und eine die Kennzeichnung erfassende Erfassungseinrichtung umfassen. Zweckmäßigerweise ist die Kennzeichnung eine Codierung. Dabei kann es sich nach einer ersten Erfindungsalternative um eine elektronische Codierung handeln, die über die Erfassungseinrichtung abfragbar ist. Auch hier kann als Erfassungseinrichtung beispielsweise die Auswerteeinrichtung selbst verwendet werden. Denkbar ist, dass jeder Strahlungsfilter über einen kleine integrierten Chip verfügt, der beispielsweise beim Einschieben automatisch mit einem entsprechenden Anschlussstecker kontaktiert wird, worüber die Verbindung zur Erfassungseinrichtung geschlossen wird.

Alternativ kann dazu die Kennzeichnung auch eine mittels einer optischen Leseeinrichtung zu erfassende Kennzeichnung sein. Denkbar ist z.B. eine Aufschrift oder ein Aufdruck, insbesondere ein Barcode oder dergleichen. Auch sind beispielsweise bestimmte Reflexionsmuster an dem Filter als Kennzeichnung verwendbar, die über die optische Leseeinrichtung erfasst werden. Eine zweckmäßige Alternative sieht dem gegenüber vor, das die Kennzeichnung eine am Strahlungsfilter angebrachte Struktur, insbesondere in Form von Kerben oder dergleichen ist. Diese Struktur kann ebenfalls über die optische Leseeinrichtung erfasst werden.

Eine weitere Erfindungsalternative zur Ausbildung der Kennzeichnung schlägt vor, dass die Kennzeichnung mittels am Strahlungsfilter vorgesehenen Vorsprünge oder Eintiefungen, z.B. in Form von Kerben, realisiert ist, über welche bei eingesetztem Strahlungsfilter Schalt- oder Sensorelemente betätigt werden. Die Vorsprünge oder Eintiefungen sind für jeden Strahlungsfilter spezifisch ausgebildet und/oder positioniert, so dass sich für jeden Strahlungsfilter eine spezifische Schalt- oder Sensorelementbetätigung aufgrund der entsprechenden Ausgestaltung ergibt. Aus der Kombination der betätigten Schalt- oder Sensorelemente ist dann der jeweilige Filtertyp oder die Filterart erkennbar.

Schließlich besteht noch die Möglichkeit, magnetische Kennzeichnungen zu verwenden, die z.B. über Hallsensoren, die das erzeugte Magnetfeld detektieren, erfasst werden können. Beispielsweise können hier längs einer Filterseite filterspezifisch mehrere magnetische Kennzeichnungen angeordnet werden, deren Positionierung oder deren Magnetfelder wiederum eine Codierung für die Filterart und/oder den Filtertyp darstellt. Je nachdem wie dann die von dem oder den Hallsensoren gelieferten Ausgangssignale sind lässt sich hierüber die Filterart oder der Filtertyp bestimmen.

Wie bereits beschrieben ist es zweckmäßig, wenn die Flächendosismesseinrichtung in einer der Strahlungsquelle nachgeschalteten Blendeneinrichtung, insbesondere der Tiefenblende integriert ist, so dass sich insgesamt ein abgeschlossenes System ergibt. Die Auswerteeinrichtung kann dabei ebenfalls integriert sein, es besteht jedoch die Möglichkeit, das diese extern positioniert ist.

Wenngleich die Möglichkeit besteht, jedem Strahlungsfilter einen spezifischen Korrekturwert zuzuordnen, der stets bei Verwendung des Strahlungsfilters zur Korrektur herangezogen wird, ist es zweckmäßig, wenn der zur Korrektur verwendete Korrekturwert von wenigstens einem ein Maß für die erzeugte Röntgenstrahlung darstellenden Parameter abhängig ist. Die applizierte Röntgenstrahlung ist in weiten Bereichen durch entsprechenden Einstellung der Betriebsparameter, also beispielsweise der Betriebsspannung oder dem Betriebsstrom variierbar. Um die Korrektur des Flächendosisproduktes noch genauer gestalten zu können, ist es zweckmäßig einen Korrekturwert zu verwenden, der auf die verwendeten, die Röntgenstrahlung beeinflussten Parameter abgestimmt ist. Das heißt, es sind letztlich für jeden Strahlungsfilter beispielsweise mehrere Korrekturwerte in der Auswerteeinrichtung abgelegt, die bestimmten die Röntgenstrahlung beeinflussenden Parametern wie beispielsweise der Röhrenspannung oder dem Röhrenstrom zugeordnet oder von diesen abhängig sind. Je nachdem welche Betriebsparameter vom Arzt oder der Röntgenassistentin eingestellt werden wählt dann die Auswerteeinrichtungen, der die entsprechenden Betriebsparameterinformationen vorliegen, den entsprechenden Korrekturwert aus der abgelegten filterspezifischen Korrekturwerteschar aus. Hierüber ist also eine die tatsächlichen Betriebsgegebenheiten berücksichtigende Korrektur möglich.

Weitere Vorteile, Merkmale und Einzelheiten der Erfindung ergeben sich aus dem im folgenden beschriebenen Ausgangsbeispiel sowie anhand der Zeichnungen:
- Fig. 1: eine Prinzipskizze einer erfindungsgemäßen Röntgeneinrichtung sowie eines lösbar einsetzbaren Strahlungsfilters, und
- Fig. 2: eine Prinzipdarstellung der relevanten Teile der Flächendosismesseinrichtung.

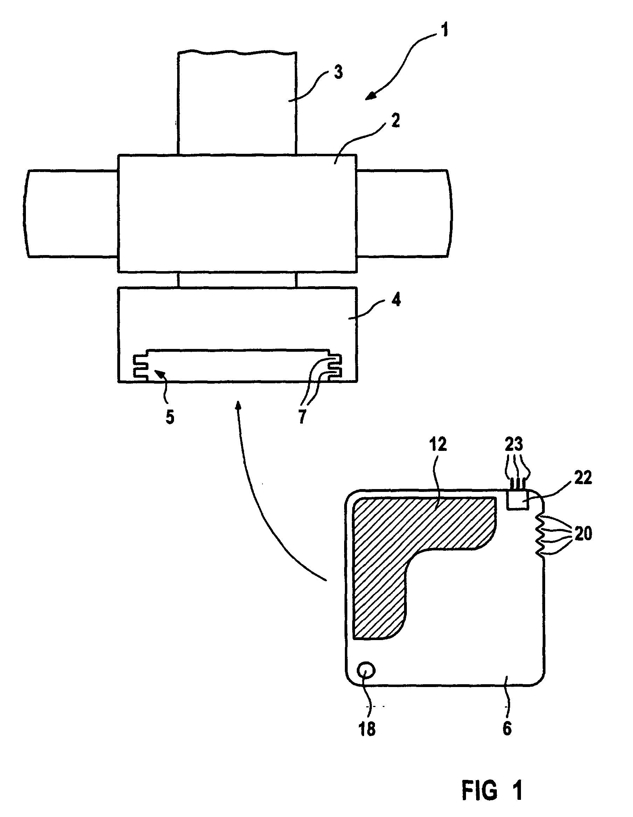

Fig. 1 zeigt eine erfindungsgemäße Röntgeneinrichtung 1, wobei hier lediglich die Strahlungsquelle 2, die an einem Teleskoparm 3 eines nicht näher gezeigten deckenmontierten Stativs angeordnet ist, gezeigt ist. Unterhalb der Strahlungsquelle 2 ist im gezeigten Beispiel eine Blendeneinrichtung 4 z.B. eine Tiefenblende vorgesehen, über die die Form des Strahls in der x- und y-Richtung beziehungsweise in der hierüber aufgespannten Ebene geformt werden kann, worauf nachfolgend noch kurz eingegangen wird.

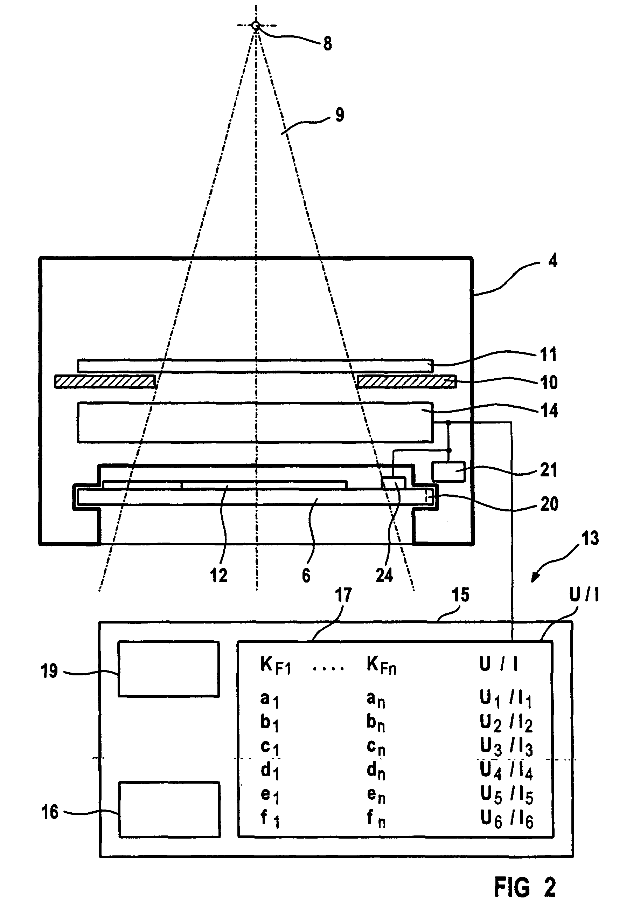

Die Tiefenblende 4 weist gehäuseseitig Aufnahmen 5 zur Aufnahme eines oder mehrerer Strahlungsfilter 6 auf. Im gezeigten Beispiel sind die Aufnahmen als seitliche Einschubrillen 7 ausgebildet, in die ein im Wesentlichen rechteckiger, plattenförmiger Strahlungsfilter 6 eingeschoben wird. Er befindet sich dann, siehe Fig. 2, im Strahlengang der von der Strahlungsquelle 2, die in Fig. 2 nicht näher gezeigt ist, erzeugten Röntgenstrahlung. Von der Strahlungsquelle 2 ist lediglich der Fokus 8 gezeigt, von dem ausgehend sich der Röntgenstrahl 9 aufweitet. Über x- und y-Blenden 10, 11 wird die Form des Röntgenstrahls definiert. Der Röntgenstrahl tritt ebenfalls durch den Strahlungsfilter 6 hindurch, wobei er in den Bereichen, in denen der Strahlungsfilter ein Röntgenstrahlenfiltermedium 12 aufweist geschwächt wird.

Um nun das Flächendosisprodukt bestimmen zu können, ist eine Flächendosismesseinrichtung 13 vorgesehen, die eine im Strahlengang angeordnete und dem Strahlungsfilter 6 vorgeschaltete Messkammer 14, in der Regel eine Ionisationskammer, sowie eine im gezeigten Beispiel hierzu externe Auswerteeinrichtung 15 umfasst. Die Röntgenstrahlung dringt in die Ionisationskammer 14 ein, was abhängig von der Strahlungsdosis zu einer Teilchenionisation und letztlich zu einem vom Ionisationsgrad abhängigen Ausgangssignal führt. Die Funktionsweise einer solchen Ionisationskammer sowie der auf das Ausgangssignal gestützten Berechnung des Flächendosisproduktes ist hinreichend bekannt. Die Berechnung erfolgt in der Auswerteeinrichtung 15, die das Ausgangssignal der Ionisationskammer 14 empfängt. Zur Berechnung ist ein geeignetes Rechenmittel 16 vorgesehen. Über die Auswerteeinrichtung 15 bzw. das Rechenmittel 16 kann, worauf nicht näher eingegangen wird, im Bedarfsfall auch eine teilweise Anlagensteuerung bei Erreichen eines hinreichenden Flächendosisproduktes etc. erfolgen. Auch dieses ist hinreichend bekannt und muss nicht mehr dargelegt werden.

Die Auswerteeinrichtung 15 verfügt ferner über eine Speichereinrichtung 17, in welcher eine Reihe von filterspezifischen Korrekturwertescharen K_{F1}.....K_{Fn} abgelegt sind. Für jeden verwendeten Strahlungsfilter 6 - es können hier die unterschiedlichsten Strahlungsfilter eingesetzt werden - existiert ein filterspezifischer Korrekturwert für das Flächendosisprodukt, dessen Korrektur deshalb erforderlich ist, da sich die Messkammer 14 vor dem Strahlungsfilter 6 befindet und infolgedessen das ionisationskammerseitige Ausgangssignal die Filterwirkung und damit die Strahlungsschwächung, hervorgerufen durch den Strahlungsfilter nicht berücksichtigt.

Um nun den richtigen Korrekturwert aus der Korrekturwerteschar auswählen zu können, ist es erforderlich, den Strahlungsfilter in seiner Art oder seinem Typ bestimmen zu können. Zu diesem Zweck sind geeignete Mittel zur Art- oder Typerkennung vorgesehen. Zunächst ist eine entsprechende Kennzeichnung jedes Strahlungsfilters 6 erforderlich. Der in Figur 1 gezeigte Strahlungsfilter 6 zeigt einige Kennzeichnungsmöglichkeiten. Zum einen ist die Verwendung eines Transponders 18 möglich, wobei jeder Strahlungsfilter einen eigenen spezifischen Transponder 18 besitzt. Beispielsweise kann in der Auswerteeinrichtung 15 eine geeignete Ansteuereinrichtung 19 vorgesehen sein, die den Transponder 18 ansteuert, so dass dieser sein Transpondersignal sendet, was von der Ansteuereinrichtung 19 wiederum empfangen und ausgewertet und auf diese Weise der entsprechende Filter erkannt werden kann. Jeder Filter verfügt über einen eigenen, ein filterspezifisches Transpondersignal aussendenden Transponder, so dass eine definitive Unterscheidung und Erkennung möglich ist.

Eine weitere alternative Kennzeichnungsmöglichkeit sieht mehrere spezifisch geformte Eintiefungen 20 am Rand des Strahlungsfilters 6 vor, die z.B. über eine optische Einrichtung 21, die in der Blendeneinrichtung 4 integriert ist, erfasst werden können. Die Kennzeichnung und Codierung des jeweiligen Filtertyps erfolgt über die Form der verwendeten Eintiefungen 20 und deren Anordnung und Positionierung zueinander. Auch hier sind beliebigste Codierungen möglich.

Alternativ besteht auch die Möglichkeit Reflexionsfelder aufzubringen, die über die optische Leselampeneinrichtung 21 erfasst werden können.

Eine weitere in Fig. 1 dargestellte Möglichkeit ist die Verwendung einer elektronischen Kennzeichnung 22 z.B. in Form eines kleinen Mikrochips, der über seine Anschlusspins 23 mit einer geeigneten blendenseitigen Erfassungseinrichtung 24 automatisch gekoppelt wird, die dann ein entsprechendes Ausgangssignal bereitstellt, das an die Auswerteeinrichtung 15 gegeben wird. Es ist an dieser Stelle darauf hinzuweisen, dass an einem Strahlungsfilter lediglich eine Kennzeichnungsmöglichkeit vorgesehen werden muss, die Verwendung der drei verschiedenen Kennzeichnungsmöglichkeiten in Fig. 1 ist hier rein exemplarisch. Entsprechendes gilt für die Verwendung der zum Erkennen der Kennzeichnung dienenden Mittel, das heißt von der Ansteuerungseinrichtung 19, dem optischen Lesemittel 21 oder dem elektronischen Lesemittel 24 ist ebenfalls jeweils nur eines vorzusehen.

In jedem Fall erhält die Auswerteeinrichtung 15 ein den Filtertyp oder die Filterart beschreibendes Informationssignal. Daneben werden der Auswerteeinrichtung 15 Informationsdaten über die zum Erzeugen der Röntgenstrahlung eingestellten Betriebsparameter Röhrenspannung U und Röhrenstrom I gegeben. Diese dienen neben der Information über den eingesetzten Filter dazu, aus der Korrekturwerteschar den richtigen filterspezifischen Korrekturwert auszuwählen.

In Fig. 2 sind im Speicherbereich 17 exemplarisch zwei Korrekturwertescharen für zwei spezifische Strahlungsfilter, nämlich die Filter F1 und die Filter Fn dargestellt. Die Korrekturwerte sind jeweils a₁, b₁.....f₁ bzw. aₙ, bₙ.....fₙ, wobei der Index 1 die Korrekturwerteschar für den Filter F1 und der Index n die Korrekturwerteschar für den Filter Fn angibt.

Ferner sind jeweils Betriebsparameter U/I angegeben, und zwar U₁/I₁, U₂/I_{2,} .....,U₆/I₆.

Anhand des anstehenden Informationssignals über den eingeschobenen Filter wird die jeweilige Korrekturwerteschar K_{F1}....K_{Fn} ausgewählt. Es sei angenommen, dass der Strahlungsfilter F1 eingeschoben sei, so dass also einer der Korrekturwerte a₁,....f₁ zur Korrektur zu verwenden sein wird.

Die genaue Bestimmung des zu verwendenden Korrekturwerts erfolgt nun anhand der anstehenden Spannungs- und Stromsignale. Es sei angenommen, dass die Spannung und der Strom in einem jeweiligen Wertbereich um U₃ und I₃ liegen. Das heißt es würde in diesem Fall der Korrekturwert c₁ zur Korrektur des ursprünglich berechneten Flächendosisproduktes ohne Berücksichtigung der Filterschwächung herangezogen. Der Korrekturwert c₁ kann beispielsweise ein definierter Wert z.B. in einer µGym²-Einheit sein, der vom berechneten Flächendosisprodukt subtrahiert wird. Alternativ kann es auch ein entsprechender Prozentsatz sein, um den das errechnete Flächendosisprodukt zu reduzieren ist etc. Denkbar sind hier unterschiedliche Korrekturwerteformen. Auch ist es denkbar, wenn die anliegenden Spannungs- und Stromwerte nicht in einem vorgegebenen Intervallbereich liegen, wenn sie also z.B. nicht U₄ und I₄ zuzuordnen sind, sondern beispielsweise U₂ und I₅, zur Ermittlung des konkreten Korrekturwerts eine bestimmte Auswahl zu treffen, so dass z.B. auch bei einer solchen Kombination C1 gewählt wird. Alternativ besteht die Möglichkeit beispielsweise einen Mittelwert aus B₁ und E₁ (bezüglich U₂ bzw. I₅) zu berechnen. Auch hier sind unterschiedliche Ansätze denkbar.

Das auf diese Weise berechnete Flächendosisprodukt wird dann beispielsweise von der Recheneinrichtung 16 entweder ausgegeben und an einem Monitor angezeigt oder aber der zentralen Steuerungseinrichtung gegeben, welches das Flächendosisproduktes im Rahmen der übergeordneten Steuerung der Recheneinrichtung berücksichtigt.

## Patentansprüche

1. Röntgeneinrichtung umfassend eine Strahlungsquelle (2), einen austauschbaren Strahlungsfilter (6) und eine Flächendosismesseinrichtung (13) umfassend eine Messkammer (14) mit zugeordneter Auswerteeinrichtung (15) zur Ermittlung des Flächendosisprodukts anhand der von der Messkammer gegebenen Messsignale, wobei die Messkammer bezogen auf die Strahlungsrichtung vor dem Filter im Strahlengang angeordnet ist, **dadurch gekennzeichnet, dass** Mittel zum Erkennen der Art und/oder des Typs des Strahlungsfilters (6) vorgesehen sind, wobei die Auswerteeinrichtung (15) zum Korrigieren des ermittelten Flächendosisprodukts anhand wenigstens eines filterspezifischen Korrekturwerts (a₁, b₁, c₁, ..., dₙ, eₙ, fₙ) ausgebildet ist.

2. Röntgeneinrichtung nach Anspruch 1, **dadurch gekennzeichnet, dass** die Mittel zum Erkennen der Art und/oder des Typs des Strahlungsfilters (6) wenigstens einen am Strahlungsfilter (6) angeordneten Transponder (18) und eine das Transpondersignal aufnehmende Erfassungseinrichtung (19) umfassen.

3. Röntgeneinrichtung nach Anspruch 1, **dadurch gekennzeichnet, dass** die Mittel zum Erkennen der Art und/oder des Typs wenigstens eine am Strahlungsfilter (6) vorgesehene die Art und/oder den Typ des Strahlungsfilters (6) angebenden Kennzeichnung und eine die Kennzeichnung erfassende Erfassungseinrichtung umfassen.

4. Röntgeneinrichtung nach Anspruch 3, **dadurch gekennzeichnet, dass** die Kennzeichnung eine Codierung ist.

5. Röntgeneinrichtung nach Anspruch 3 oder 4, **dadurch gekennzeichnet, dass** die Kennzeichnung eine elektronische Kennzeichnung (22) ist, die über die Erfassungseinrichtung (24) abfrag- oder auslesbar ist.

6. Röntgeneinrichtung nach Anspruch 3 oder 4, **dadurch gekennzeichnet, dass** die Kennzeichnung eine mittels einer optischen Leseseinrichtung (21) zu erfassende Kennzeichnung ist.

7. Röntgeneinrichtung nach Anspruch 6, **dadurch gekennzeichnet, dass** die Kennzeichnung eine Aufschrift oder ein Aufdruck, insbesondere ein Barcode o.dgl. ist.

8. Röntgeneinrichtung nach Anspruch 6, **dadurch gekennzeichnet, dass** die Kennzeichnung eine am Strahlungsfilter (6) angebrachte Struktur, insbesondere in Form von Kerben (20) o.dgl. ist.

9. Röntgeneinrichtung nach Anspruch 3 oder 4, **dadurch gekennzeichnet, dass** die Kennzeichnung mittels am Strahlungsfilter vorgesehener Vorsprünge oder Eintiefungen realisiert ist, mittels welchen bei eingesetztem Strahlungsfilter Schalt- oder Sensorelemente betätigt werden.

10. Röntgeneinrichtung nach Anspruch 3 oder 4, **dadurch gekennzeichnet, dass** die Kennzeichnung magnetisch ist.

11. Röntgeneinrichtung nach einem der vorangehenden Ansprüche, **dadurch gekennzeichnet, dass** die Flächendosismesseinrichtung (13) zumindest zum Teil in einer der Strahlungsquelle (2) nachgeschalteten Blendeneinrichtung (4), insbesondere der Tiefenblende integriert ist.

12. Röntgeneinrichtung nach einem der vorangehenden Ansprüche, **dadurch gekennzeichnet, dass** der zur Korrektur verwendete Korrekturwert (a₁, b₁, c₁, ...,dₙ, eₙ, fₙ) von wenigstens einem ein Maß für die erzeugte Röntgenstrahlung darstellenden Parameter abhängig ist.

13. Röntgeneinrichtung nach Anspruch 12, **dadurch gekennzeichnet, dass** der Korrekturwert (a₁, b₁, c₁, ..., dₙ, eₙ, fₙ) von der Betriebsspannung (U) und/oder dem Betriebsstrom (I) der Strahlungsquelle (2) abhängig ist.

14. Röntgeneinrichtung nach Anspruch 12 oder 13, **dadurch gekennzeichnet, dass** in der Auswerteeinrichtung (15) eine Schar parameterbezogener Korrekturwerte abgelegt sind, aus welcher der zur Korrektur zu verwendende parameterabhängige Korrekturwert (a₁, b₁, c₁, ..., dₙ, eₙ, fₙ) gewählt wird.

## Claims

1. X-ray apparatus comprising a radiation source (2), an interchangeable radiation filter (6) and an area dose measuring device (13) with a measurement chamber (14) and an allocated evaluation device (15) for determining the area dose product on the basis of the measured signals provided by the measurement chamber, with the measurement chamber arranged preceding the filter in the beam path with reference to the radiation propagation direction,
**characterised in that**
means are provided for detecting the nature and/or type of radiation filter (6), whereby an evaluation device (15) fashioned for correcting the calculated surface dose product on the basis of at least one filter-specific correction value (*a*₁,*b*₁,*c*₁.....,*d*_{*n*},*e*_{*n*},*f*_{*n*}).

2. X-ray apparatus according to Claim 1, **characterised in that**
the detector for detecting the nature and/or type of the radiation filter (6) comprise at least one transponder (18) arranged at the radiation filter (6) and an acquisition device (19) that picks up the transponder signal.

3. X-ray apparatus according to Claim 1, **characterised in that** the detector for detecting the nature and/or type of at least one identification specifying the nature and/or type of the filter provided at the radiation filter (6) and an acquisition device that acquires the identification.

4. X-ray apparatus according to Claim 3, **characterised in that** the identification is a coding.

5. X-ray apparatus according to Claim 3 or 4
**characterised in that**
the identification is an electronic identification (22), which can be interrogated or read out by said acquisition device (24).

6. X-ray apparatus according to Claim 3 or 4,
**characterised in that**
the identification is an identification which is to be detected by means of an optical reading device (21).

7. X-ray apparatus according to Claim 6, **characterised in that** the identification is a label or an imprint, in particular a barcode or the like.

8. X-ray apparatus according to Claim 6,
**characterised in that**
the identification is a structure applied to the radiation filter (6), in particular in the form of notches (20) or the like.

9. X-ray apparatus according to Claim 3 or 4, **characterised in that** q the identification is realised by means of projections or indentations provided at said radiation filter, by means of which switches and sensor elements are actuated with the radiation filter used.

10. X-ray apparatus according to Claim 3 or 4,
**characterised in that** the identification is magnetic.

11. X-ray apparatus according to one of the preceding Claims,
**characterised in that** the surface dose measurement device (13) is at least partially integrated into a diaphragm device (4) arranged downstream of the radiation source (2), in particular the depth diaphragm.

12. X-ray apparatus according to one of the preceding Claims,
**characterised in that** the correction value (*a*₁,*b*₁,*c*₁,....*d*_{*n*},*e*_{*n*},*f*_{*n*},) employed for correction is dependent on at least one parameter displaying a measure for the generated X-rays.

13. X-ray apparatus according to Claim 12,
**characterised in that** the correction value (*a*₁,*b*₁,*c*₁,....*d*_{*n*},*e*_{*n*},*f*_{*n*},) is dependent on the operating voltage (U) and the operating current (I) of the radiation source (2).

14. X-ray apparatus according to Claim 12 or 13 **characterized in that** a family of parameter-related correction values are distributed in the evaluation device (15) from which the parameter-dependent correction value (*a*₁,*b*₁,*c*₁,....*d*_{*n*},*e*_{*n*},*f*_{*n*},) to be employed for correction is selected.

## Revendications

1. Dispositif à rayons X, comprenant source (2) de rayonnement, un filtre (6) pour du rayonnement, qui peut être remplacé, et un dispositif (13) de mesure de dose en surface comprenant une chambre (14) de mesure ayant un dispositif (15) d'interprétation associé, pour déterminer le produit de dose en surface, au moyen de signaux de mesure donnés par la chambre de mesure, la chambre de mesure étant disposée par rapport au sens du rayonnement en amont du filtre dans le trajet des rayons, **caractérisé en ce qu'**il est prévu des moyens de détection de la nature et/ou du type du filtre (6) pour du rayonnement, le dispositif (15) d'interprétation étant constitué pour corriger le produit de dose en surface déterminé au moyen d'au moins une valeur (a₁, b₁, c₁, ..., dₙ, eₙ, fₙ) de correction spécifique au filtre.

2. Dispositif à rayons X suivant la revendication 1, **caractérisé en ce que** les moyens de détection de la nature et/ou du type du filtre (6) pour du rayonnement comprennent au moins un transpondeur (18) disposé sur le filtre (6) pour du rayonnement et un dispositif (19) de détection recevant le signal du transpondeur.

3. Dispositif à rayons X suivant la revendication 1, **caractérisé en ce que** les moyens de détection de la nature et/ou du type comprennent au moins une caractéristique prévue sur le filtre (6) pour du rayonnement et indiquant la nature et/ou le type du filtre (6) pour du rayonnement et un dispositif de détection détectant la caractéristique.

4. Dispositif à rayons X suivant la revendication 3, **caractérisé en ce que** la caractéristique est un code.

5. Dispositif à rayons X suivant la revendication 3 ou 4, **caractérisé en ce que** la caractéristique est une caractéristique (22) électronique, qui peut être interrogée ou lue par le dispositif (24) de détection.

6. Dispositif à rayons X suivant la revendication 3 ou 4, **caractérisé en ce que** la caractéristique est une caractéristique à détecter au moyen d'un dispositif de lecture optique.

7. Dispositif à rayons X suivant la revendication 6, caractérisé ne ce que la caractéristique est apposée par l'écriture ou par impression, en étant notamment un code barre ou analogue.

8. Dispositif à rayons X suivant la revendication 6, **caractérisé en ce que** la caractéristique est une structure placée sur le filtre (6) pour du rayonnement, notamment sous la forme d'encoches (20) ou analogue.

9. Dispositif à rayons X suivant la revendication 3 ou 4, **caractérisé en ce que** la caractéristique est réalisée au moyen de saillies ou de creux prévus sur le filtre pour du rayonnement, au moyen desquels des éléments de commutation ou des capteurs peuvent être actionnés lorsque le filtre pour du rayonnement est utilisé.

10. Dispositif à rayons X suivant la revendication 3 ou 4, **caractérisé en ce que** la caractéristique est magnétique.

11. Dispositif à rayons X suivant l'une des revendications précédentes, **caractérisé en ce que** le dispositif (13) de mesure de dose en surface est intégré au moins en partie dans un dispositif (4) à diaphragme monté en aval de la source (2) de rayonnement, notamment dans le diaphragme de profondeur.

12. Dispositif à rayons X suivant l'une des revendications précédentes, **caractérisé en ce que** la valeur (a₁, b₁, c₁, ..., dₙ, eₙ, fₙ) utilisée pour la correction dépend d'au moins un paramètre représentant une mesure du rayonnement X produit.

13. Dispositif à rayons X suivant la revendication 12, **caractérisé en ce que** la valeur (a₁, b₁, c₁, ..., dₙ, eₙ, fₙ) de correction dépend de la tension (U) de fonctionnement et/ou du courant (I) de fonctionnement de la source (2) de rayonnement.

14. Dispositif à rayons X suivant la revendication 12 ou 13, **caractérisé en ce que** dans le dispositif (15) d'interprétation est mémorisée une famille de valeurs de correction rapportée à des paramètres dans lesquels on choisit la valeur (a₁, b₁, c₁, ..., dₙ, eₙ, fₙ) de correction en fonction du paramètre à utiliser pour la correction.
